Europäisches Patentamt

European Patent Office

Office européen des brevets

(11) Veröffentlichungsnummer: **0 287 014**
**A2**

## (12) EUROPÄISCHE PATENTANMELDUNG

(21) Anmeldenummer: 88105731.9

(22) Anmeldetag: 11.04.88

(51) Int. Cl.⁴: **A61K 31/685 , A61K 31/575**

Die Bezeichnung der Erfindung wurde geändert (Richtlinien für die Prüfung im EPA, A-III, 7.3).

Patentansprüche für folgende Vertragsstaaten: ES + GR.

(30) Priorität: 15.04.87 CH 1480/87

(43) Veröffentlichungstag der Anmeldung: 19.10.88 Patentblatt 88/42

(84) Benannte Vertragsstaaten: AT BE CH DE ES FR GB GR IT LI LU NL SE

(71) Anmelder: **F. HOFFMANN-LA ROCHE & CO. Aktiengesellschaft**

**CH-4002 Basel(CH)**

(72) Erfinder: **Cassal, Jean-Marie, Dr.
rue du Markstein 4
F-68100 Mulhouse(FR)**
Erfinder: **Lengsfeld, Hans, Dr.
Unterer Rebbergweg 96
CH-4153 Reinach(CH)**

(74) Vertreter: **Lederer, Franz, Dr. et al
Patentanwält Dr. Franz Lederer Lucile
Grahnstrasse 22
D-8000 München 80(DE)**

(54) **Cholesteryl-phosphorylcholin zur Senkung des Cholesterinspiegels.**

(57) Die Erfindung betrifft das Cholesteryl-phosphorylcholin und pharmazeutisch annehmbare Salze davon als pharmazeutische, insbesondere die Cholesterinabsorption hemmende und das Plasmacholesterin senkende Wirkstoffe, sowie Arzneimittel enthaltend Cholesteryl-phosphorylcholin oder ein pharmazeutisch annehmbares Salz davon.

EP 0 287 014 A2

## Verwendung eines Steroids

Im Rahmen der vorliegenden Erfindung wurde gefunden, dass das aus J. Org. Chem. 43, 1978, 2331 bekannte Steroid Cholesteryl-phosphorylcholin (im folgenden CPC) sowie die pharmazeutisch annehmbaren Salze davon wertvolle physiologische Eigenschaften haben, indem sie eine die Cholesterinabsorption hemmende und das Plasmacholesterin senkende Wirkung aufweisen. Die vorliegende Erfindung betrifft demnach CPC und die oben erwähnten Salze als pharmazeutische Wirkstoffe, ferner Arzneimittel enthaltend CPC oder eines dieser Salze und die Herstellung solcher Arzneimittel, sowie die Verwendung von CPC und dessen Salzen bei der Bekämpfung bzw. Verhütung von Krankheiten, insbesondere der Hypercholesterinämie und der Atherosklerose, bzw. die Verwendung von CPC und dessen Salzen zur Herstellung von Arzneimitteln für die Bekämpfung bzw. Verhütung dieser Krankheiten.

Beispiele von pharmazeutisch annehmbaren Salzen von CPC sind Salze anorganischer Säuren, wie das Hydrochlorid oder das Sulfat; Salze organischer Säuren, wie das Mesylat oder Tosylat; und Salze mit Basen, wie das Natriumsalz.

Die weiter oben erwähnten physiologischen Eigenschaften von CPC lassen sich tierexperimentell wie folgt nachweisen:

Einer Gruppe von 12 Totenkopfäffchen wurde ein Gemisch enthaltend Triolein, Stärke, Milchpulver, 1 mg [$^{14}$C]-Cholesterin und 0.05 mg [$^{3}$H]-Polyäthylenglycol und unmittelbar darauf eine Dispersion von CPC in 5% Gummi arabicum oral verabreicht. Hierauf wurde der Kot während 2,5 Tagen gesammelt. Die ausgeschiedene [$^{14}$C]-Radioaktivität des Cholesterins wurde unter Berücksichtigung der [$^{3}$H]-Radioaktivität des internen Standards Polyäthylenglycol ermittelt. Es wurde die Cholesterinabsorption (Differenz zwischen der verabreichten und der ausgeschiedenen [$^{14}$C]-Radioaktivität des Cholesterins) berechnet und in der nachfolgenden Tabelle in Prozenten von unbehandelten Kontrolltieren angegeben.

| Dosis ($\mu$Mol/kg) | Cholesterinabsorption (%) |
|---|---|
| 30 | 90 ± 17 |
| 100 | 60 ± 10 |
| 300 · | 12 ± 6 |

Totenkopfäffchen wurde pro Tier und Tag 239 mg Lactose als Placebo oder 239 mg CPC zusammen mit Kokonussöl enthaltendem Futter während 14 Tagen verabreicht. Jede 3 oder 4 Tage wurde den Tieren venöses Blut entnommen und der Cholesterinspiegel im Blutplasma ermittelt. In der nachstehenden Tabelle wird der Cholesterinspiegel in Prozenten der Vorperiode sowie die Senkung des Cholesterinspiegels in Prozenten der Kontrolltieren angegeben.

| Tag | 1 vor der Behandlung | 4 | 7 während der Behandlung | 11 | 14 | 18 nach der Behandlung | 21 |
|---|---|---|---|---|---|---|---|
| Placebo (%) | 100± 6 | 97±8 | 104±8 | 106±12 | 104± 7 | 103± 9 | 107± 8 |
| CPC (%) | 100±8 | 88±6 | 84±4 | 93±13 | 86±11 | 94±13 | 97±11 |
| Senkung (%) | 0 | 9 | 20 | 13 | 18 | 9 | 10 |

Daraus geht hervor, dass CPC eine reversible Senkung des Plasmacholesterols von 10 bis 20% gegenüber der Placebowerte und der Werte in der Vorperiode aufweist.

Was die Toxizität anbelangt, beträgt die $LD_{50}$ von CPC 125 mg/kg i.v. und 4000 mg/kg p.o. an der Maus.

CPC und dessen pharmazeutisch annehmbare Salze können als Heilmittel, z.B in Form pharmazeutischer Präparate, Verwendung finden. Die pharmazeutischen Präparate werden oral, z.B. in Form von Tabletten, Lacktabletten, Dragées, Hart-und Weichgelatinekapseln, Lösungen, Emulsionen oder Suspensionen, verabreicht.

Zur Herstellung von pharmazeutischen Präparaten können diese Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze verwenden. Für Weichgelatinekapseln eignen sich als Träger beispielsweise pflanzliche Oele, Wachse, Fette, halbfeste und flüssige Polyole; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker und Glukose.

Die Pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten Sie können auch noch andere therapeutisch wertvolle Stoffe enthalten.

Wie eingangs erwähnt sind Arzneimittel, enthaltend CPC oder ein pharmazeutisch annehmbares Salz davon ebenfalls Gegenstand der vorliegenden Erfindung, weiterhin auch ein Verfahren zur Herstellung solcher Arzneimittel, welches dadurch gekennzeichnet ist, dass man eine oder mehrere dieser Produkte und gegebenenfalls einen oder mehrere andere therapeutisch wertvolle Stoffe in eine galenische Darreichungsform bringt.

Wie eingangs erwähnte können die genannten Produkte bei der Bekämpfung bzw. Verhütung von Krankheiten verwendet werden. Man kann sie insbesondere bei der Bekämpfung bzw. Verhütung der Hypercholesterinämie und der Atherosklerose verwenden. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 50 mg bis etwa 3 g, vorzugsweise von etwa 200 mg bis etwa 1 g angemessen sein.

Beispiel

Man kann CPC oder ein pharmazeutisch annehmbares Salz davon wie folgt als Wirkstoff zur Herstellung von pharmazeutischen Präparaten verwenden:

| a)  Tabletten | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10-proz. Lösung von Hydroxypropylmethylcellulose in einem Gemisch aus Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

| b)  Kapseln | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

**Ansprüche**

1. Das Cholesteryl-phosphorylcholin und pharmazeutisch annehmbare Salze davon als pharmazeutische, insbesondere die Cholesterinabsorption hemmende und das Plasmacholesterin senkende Wirkstoffe.

2. Arzneimittel, enthaltend Cholesteryl-phosphorylcholin oder ein pharmazeutisch annehmbares Salz davon, insbesondere zur Hemmung der Cholesterinabsorption und zur Senkung des Plasmacholesterins.

3. Verwendung des Cholesteryl-phosphorylcholins oder eines pharmazeutisch annehmbaren Salzes davon bei der Bekämpfung bzw. Verhütung von Krankheiten, insbesondere Hypercholesterinämie und Atherosklerose.

4. Verwendung des Cholesteryl-phosphorylcholins oder eines pharmazeutisch annehmbaren Salzes davon zur Herstellung von Arzneimitteln für die Bekämpfung bzw. Verhütung von Krankheiten, insbesondere Hypercholesterinämie und Atherosklerose.

Patentansprüche für folgende Vertragsstaaten: GR: ES

1. Verfahren zur Herstellung von Arzneimitteln enthaltend Cholesteryl-phosphorylcholin oder ein pharmazeutisch annehmbares Salz davon, insbesondere zur Hemmung der Cholesterinabsorption und zur Senkung des Plasmacholesterins, dadurch gekennzeichnet. dass man das Cholesteryl-phosphorylcholin oder ein pharmazeutisch annehmbares Salz davon mit einem oder mehreren therapeutisch inerten Excipientien in eine galenische Darreichungsform bringt.

2. Verwendung des Cholesteryl-phosphorylcholins oder eines pharmazeutisch annehmbaren Salzes davon zur Herstellung von Arzneimitteln für die Bekämpfung bzw. Verhütung von Krankheiten, insbesondere Hypercholesterinämie und Atherosklerose.